# EUROPEAN PATENT SPECIFICATION

(11) **EP 3 432 777 B1**
(45) Date of publication and mention of the grant of the patent: **04.09.2024**
(21) Application number: 17718632.7
(22) Date of filing: 16.03.2017
(51) Int. Cl.: A61B 5/00, A61B 5/02, A61B 5/0215

(54) **MICROCATHETER SENSOR DESIGN FOR MOUNTING SENSOR TO MINIMIZE INDUCED STRAIN**
MIKROKATHETERSENSORDESIGN ZUR MONTAGE EINES SENSORS ZUR MINIMIERUNG VON INDUZIERTER BELASTUNG
CONCEPT DE CAPTEUR À MICROCATHÉTER POUR LE MONTAGE DE CAPTEUR POUR MINIMISER LA DÉFORMATION INDUITE

(30) Priority: 23.03.2016 US 201615077964
(43) Date of publication of application: 30.01.2019
(73) Proprietor: Medtronic Vascular Inc., Santa Rosa, CA 95403 (US)
(72) Inventor: MCCAFFREY, Gerry, Santa Rosa, CA 95403 (US); MURPHY, Christopher, Santa Rosa, CA 95403 (US); SWEENEY, Fiachra, Santa Rosa, CA 95403 (US); KELLY, John, Santa Rosa, CA 95403 (US)
(74) Representative: Zimmermann & Partner Patentanwälte mbB
(86) International application number: PCT/US2017/022637
(87) International publication number: WO 2017/165179

(56) References cited:
- EP-A1- 1 498 068
- EP-A2- 1 165 171
- US-A1- 2015 359 438

## Description

### FIELD OF THE INVENTION

The invention relates to methods and systems for determining a pressure gradient across a lesion of a vessel for calculating a Fractional Flow Reserve.

### BACKGROUND OF THE INVENTION

The severity of a stenosis or lesion in a blood vessel may be assessed by obtaining proximal and distal pressure measurements relative to the given stenosis and using those measurements for calculating a value of the Fractional Flow Reserve (FFR). FFR is defined as the ratio of a first pressure measurement (P_{d}) taken on the distal side of the lesion and to a second pressure measurement taken on the proximal side of the lesion usually within the aorta (Pₐ). Conventionally, a sensor is placed on the distal portion of a guidewire or FFR wire to obtain the first pressure measurement P_{d}, while an external pressure transducer is fluidly connected via tubing to a guide catheter for obtaining the second or aortic (AO) pressure measurement Pₐ. Calculation of the FFR value provides a lesion specific index of the functional severity of the stenosis in order to determine whether the blockage limits blood flow within the vessel to an extent that treatment is needed. An optimal or normal value of FFR in a healthy vessel is 1.00, while values less than about 0.80 are generally deemed significant and in need of an interventional treatment. Common interventional treatment options include balloon angioplasty and/or stent implantation.

If an interventional treatment is required, the interventional device, such as a balloon catheter, is tracked over a guide wire to the site of the lesion. Conventional FFR wires generally are not desired by clinicians to be used as guide wires for such interventional devices. Accordingly, if an intervention treatment is required, the clinician generally removes the FFR wire, inserts a conventional guide wire, and tracks the interventional device to the treatment site over the conventional guide wire.

The mounting of a pressure sensor on the distal end of a catheter, such as a microcatheter makes it difficult to isolate the pressure sensor from bending stresses experienced as a result of interaction between the pressure sensor and the housing of the catheter. Due to the high sensitivity and size of the pressure sensor used in this application, any stress placed on the pressure sensor can cause a distortion of the sensor resulting in an incorrect pressure reading or bend error. Accordingly, there remains a need for a microcatheter to obtain pressure measurements suitable for use in calculating an FFR value for a given stenosis, whereby the clinician may use a conventional or preferential guidewire instead of a FFR guidewire. In addition, there remains a need for a FFR microcatheter to minimize both the profile of the catheter and the bending stresses experienced by the pressure sensor. Various devices measuring the pressure inside a blood vessel are known from documents US 2015/0359438 A1, EP 1 498 068 A1, and in EP 1 165 171 A2.

### BRIEF SUMMARY OF THE INVENTION

Embodiments hereof relate to a catheter, such as a pressure measurement catheter, including an elongate shaft having a proximal end optionally coupled to a handle or luer fitting and a distal end having a distal opening. The elongate shaft further includes a proximal portion, an intermediate portion, and a distal portion having a distal tip. In the proximal portion of the elongated shaft, a shaft wall may define two separate lumens: a guide wire lumen and a second or pressure sensor wire lumen, extending parallel to each other or side-by-side along the proximal portion. The distal portion of the elongate shaft is configured to receive a guidewire in a distal portion of guidewire lumen thereof. The pressure sensing wire may extend to the distal portion of the elongate shaft to be coupled to a pressure sensor mounted to a deformable member on the distal tip for measuring a pressure of a fluid within lumen of vessel. The deformable member has adhesive properties and is disposed between the pressure sensor and the catheter. The deformable member reduces the amount of stress and strain transferred to the pressure sensor, thereby reducing distortion of the sensor that may result in an incorrect pressure reading.

Embodiments hereof also relate to a catheter, such as a measurement catheter, including an elongate shaft having a proximal end optionally coupled to a handle or luer fitting and a distal end having a distal opening. The elongate shaft further includes a proximal portion, an intermediate portion, and a distal portion having a distal tip. In the proximal portion of elongated shaft, shaft wall may define two separate lumens: a guide wire lumen and a second or pressure sensor wire lumen, extending parallel to each other or side-by-side along the proximal portion. The distal portion of the elongate shaft is configured to receive a guidewire in a distal portion of the guidewire lumen thereof. The pressure sensing wire lumen may extend to the distal portion of the elongate shaft to be coupled to a pressure sensor mounted to an intermediate member on the distal tip for measuring a pressure of a fluid within lumen of vessel. The intermediate member is disposed between the pressure sensor and the catheter. The intermediate member has a first portion, which is coupled to pressure sensor and a second portion, which is coupled to the catheter. The intermediate member also has a hinge disposed between the first and second portions. When the distal portion of the catheter bends away from the pressure sensor, the catheter does not contact the intermediate member or the pressure sensor because the intermediate member maintains the pressure sensor is a substantially straight configuration relative to the bending catheter. When the distal portion of the catheter bends towards the pressure sensor, the catheter applies a force on the intermediate member. As a result of this force, the first portion of the intermediate member rotates about the hinge in the same direction as the applied force. Since the intermediate member moves the pressure sensor away from the bending catheter, the amount of stress and strain transferred to the pressure sensor is reduced, thereby reducing distortion of the sensor that may result in an incorrect pressure reading.

Embodiments hereof also relate to a catheter, such as a measurement catheter, including an elongate shaft having a proximal end optionally coupled to a handle or luer fitting and a distal end having a distal opening. The elongate shaft further includes a proximal portion, an intermediate portion, and a distal portion having a distal tip. In the proximal portion of elongated shaft, shaft wall may define two separate lumens: a guide wire lumen and a second or pressure sensor wire lumen, extending parallel to each other or side-by-side along the proximal portion. The distal portion of the elongate shaft is configured to receive a guidewire in a distal portion of the guidewire lumen thereof. The pressure sensing wire lumen may extend to the distal portion of the elongate shaft to be coupled to a pressure sensor on the distal tip for measuring a pressure of a fluid within lumen of vessel. Although the pressure sensor is coupled to the distal tip of the catheter, the pressure sensor is suspended above the shaft wall of the catheter by a step extending from the shaft wall. An electrical interconnect (or other wiring) is mounted on the step such that a distal end of the electrical interconnect extends past the step and is disposed within a pocket on the distal tip of the catheter. The pressure sensor is coupled to the distal end of the electrical interconnect and is thereby disposed within the pocket without contacting the shaft wall or the side walls of the pocket. Since the pressure sensor has no contact with the catheter, the amount of stress and strain transferred to the pressure sensor is reduced or even eliminated, thereby reducing distortion of the sensor that may result in an incorrect pressure reading.

### BRIEF DESCRIPTION OF DRAWINGS

The foregoing and other features and advantages of the invention will be apparent from the following description of embodiments hereof as illustrated in the accompanying drawings. The accompanying drawings, which are incorporated herein and form a part of the specification, further serve to explain the principles of the invention and to enable a person skilled in the pertinent art to make and use the invention. The drawings are not to scale.
FIG. 1 is a broken view of a system for measuring FFR with a distal portion thereof shown within a vessel including a lesion, the system including a measurement catheter including a pressure sensor and a guidewire, in accordance with an embodiment hereof.
FIG. 2 is a broken view of the catheter of FIG. 1 in partial longitudinal cross-section.
FIG. 3 is a cross-sectional view of the catheter taken along line 3-3 of FIG. 2.
FIG. 4 is a longitudinal cross-sectional view of the distal portion of the catheter of FIG. 1.
FIG. 5 is a longitudinal cross-sectional view of another embodiment of the distal portion of the catheter of FIG. 1.
FIG. 6A is a longitudinal cross-sectional view of another embodiment of the distal portion of the catheter of FIG. 1.
FIG. 6B is an exemplary representation of the distal portion of FIG. 6A under induced stress and strain in the direction of arrow A.
FIG. 6C is an exemplary representation of the distal portion of FIG. 6A under induced stress and strain in the direction of arrow B.
FIG. 7A is a longitudinal cross-sectional view of another embodiment of the distal portion of the catheter of FIG. 1.
FIG. 7B is a top view of the distal portion of the catheter of FIG. 7A.

### DETAILED DESCRIPTION OF THE INVENTION

Specific embodiments of the present invention are now described with reference to the figures, wherein like reference numbers indicate identical or functionally similar elements. While the disclosure refers to illustrative embodiments for particular applications, it should be understood that the disclosure is not limited thereto. Modifications can be made to the embodiments described herein without departing from the scope of the present disclosure. Those skilled in the art with access to this disclosure will recognize additional modifications, applications, and embodiments within the scope of this disclosure and additional fields in which the disclosed examples could be applied. Therefore, the following detailed description is not meant to be limiting. Further, it is understood that the systems and methods described below can be implemented in many different embodiments of hardware. Any actual hardware described is not meant to be limiting. The operation and behavior of the systems and methods presented are described with the understanding that modifications and variations of the embodiments are possible given the level of detail presented.

References to "an example," "one embodiment," "an embodiment," "in certain embodiments," etc., indicate that the embodiment described may include a particular feature, structure, or characteristic, but every embodiment may not necessarily include the particular feature, structure, or characteristic. Moreover, such phrases are not necessarily referring to the same embodiment. Further, when a particular feature, structure, or characteristic is described in connection with an embodiment, it is submitted that it is within the knowledge of one skilled in the art to affect such feature, structure, or characteristic in connection with other embodiments whether or not explicitly described.

Specific embodiments of the present invention are now described with reference to the figures, wherein like reference numbers indicate identical or functionally similar elements. The terms "distal" and "proximal" are used in the following description with respect to a position or direction relative to the treating clinician. "Distal" and "distally" are positions distant from or in a direction away from the clinician. "Proximal" and "proximally" are positions near or in a direction toward the clinician.

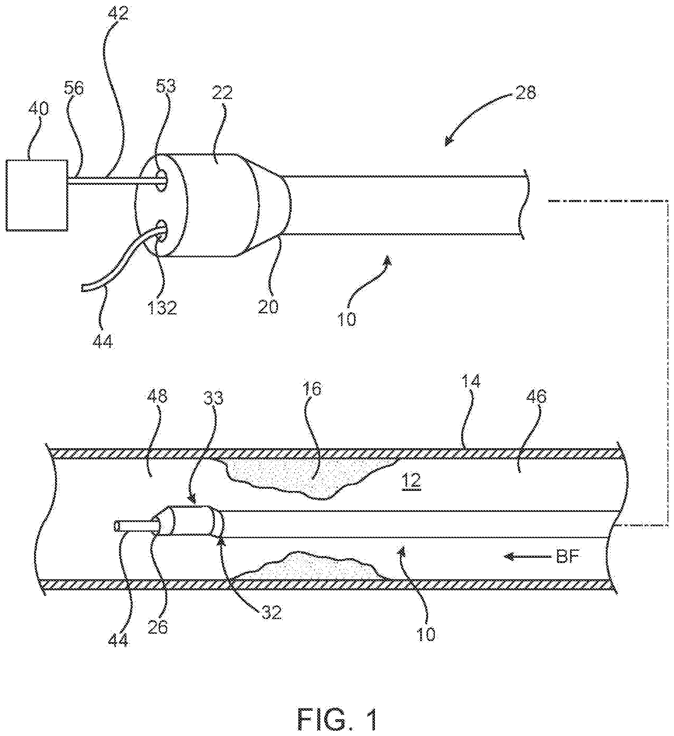

With reference to FIG. 1, a pressure measurement catheter 10 is shown with a proximal portion thereof outside of a patient and a distal portion thereof positioned *in situ* within a lumen 12 of a patient vessel 14 having a stenosis or lesion 16. In an embodiment hereof, the vessel 14 is a blood vessel such as but not limited to a coronary artery. Lesion 16 is generally representative of any blockage or other structural arrangement that results in a restriction to the flow of fluid through lumen 12 of vessel 14. Lesion 16 may be a result of plaque buildup, including without limitation plaque components such as fibrous, fibro-lipidic (fibro fatty), necrotic core, calcified (dense calcium), blood, fresh thrombus, and mature thrombus. Generally, the composition of lesion will depend on the type of vessel being evaluated. In that regard, it is understood that embodiments hereof are applicable to various types of blockage or other narrowing of a vessel that results in decreased fluid flow.

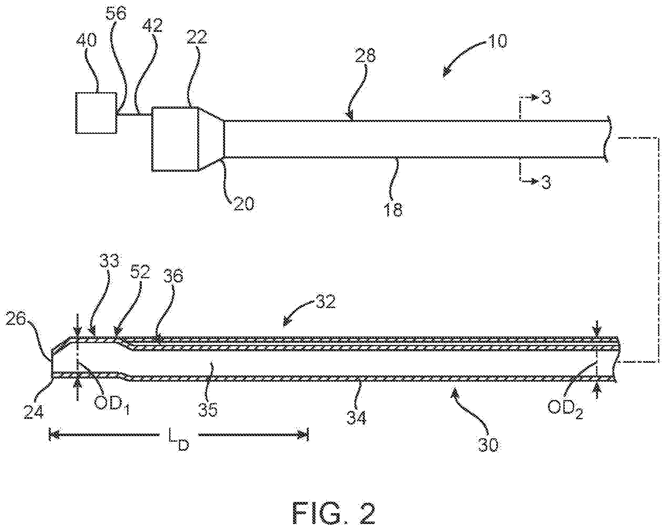

Measurement catheter 10 is shown in FIG. 2 with a distal portion thereof in longitudinal cross-section. Measurement catheter 10 includes an elongate shaft 18 having a proximal end 20 that may be coupled to a handle or luer fitting 22 and a distal end 24 having a distal opening 26. Elongate shaft 18 further includes a proximal portion 28, an intermediate portion 30, and a distal portion 32 having a distal tip 33. Although proximal portion 28, intermediate portion 30, and distal portion 32 of elongate shaft 18 have been described separately, they are described in such a manner for convenience and elongate shaft 18 may be constructed unitarily such that the portions described are part of a unitary shaft. However, different portions of elongate shaft 18 may also be constructed separately and joined together.

In embodiments hereof, elongate shaft 18 or component and/or segments thereof may be formed of polymeric materials, non-exhaustive examples of which include polyethylene terephthalate (PET), polypropylene, polyethylene, polyether block amide copolymer (PEBA), polyamide, fluoropolymers, and/or combinations thereof, either laminated, blended or coextruded. Optionally, the catheter shaft or some portion thereof may be formed as a composite having a reinforcement material incorporated within a polymeric body in order to enhance strength and/or flexibility. Suitable reinforcement layers include braiding, wire mesh layers, embedded axial wires, embedded helical or circumferential wires, and the like. In one embodiment, for example, at least a proximal portion of elongate shaft 18 may be formed from a reinforced polymeric tube. In other embodiments of an elongate tubular shaft or component in accordance herewith, a proximal segment thereof may be a hypotube of a medical grade stainless steel with outer and inner tubes of a distal segment thereof being formed from any of the polymeric materials listed above.

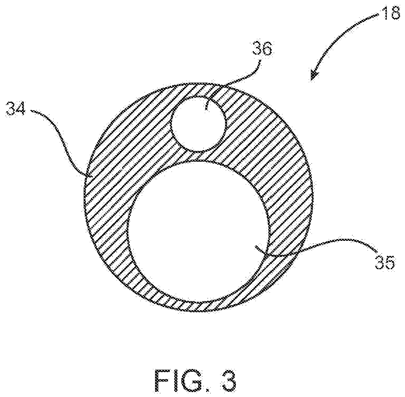

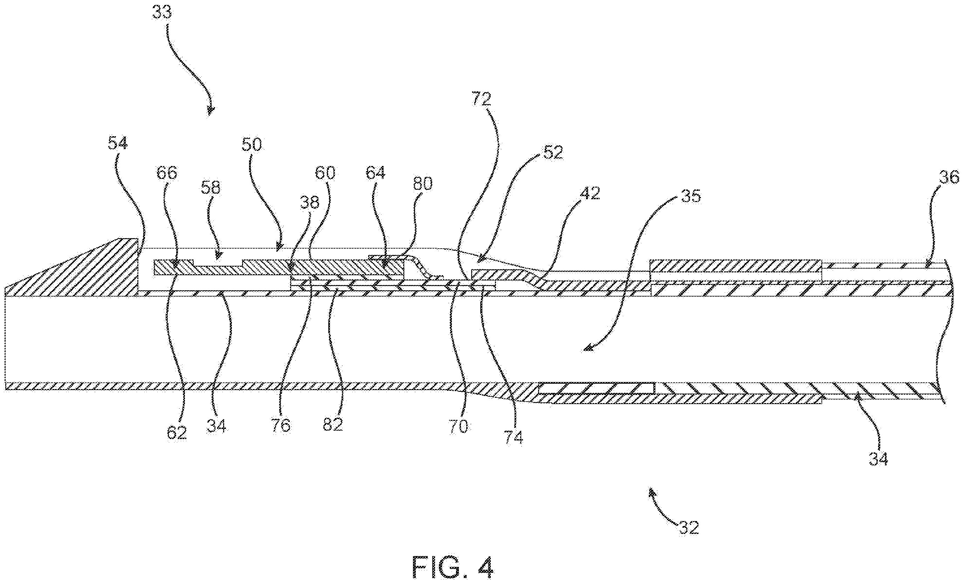

As shown in FIGS. 2-3, elongate shaft 18 has a shaft wall 34 defining a guide wire lumen 35 extending therethrough. Guide wire lumen 35 extends through proximal portion 28, intermediate portion 30, and distal portion 32. However, instead of the over-the-wire configuration shown in FIGS. 1-3, catheter 10 may have a rapid exchange configuration wherein guide wire lumen 35 extends through distal portion 32 and intermediate portion 30, and the guidewire exits shaft 18 through a rapid exchange port (not shown) in proximal portion 28, as would be understood by those skilled in the art. In one embodiment, with reference to the cross-sectional view of FIG. 3 (taken along line 3-3 of FIG. 2), in proximal portion 28 of elongated shaft 18, shaft wall 34 defines two separate lumens, guide wire lumen 35 and a second or pressure sensor wire lumen 36, extending parallel to each other or side-by-side along proximal portion 28. Communication wires 42 are omitted in FIG. 3 for clarity. Although depicted as circular in cross-section, one or more lumen(s) of elongated shaft 18 may have any suitable cross-section including for example circular, elliptical, rectangular or crescent-shaped. As explained in more detail below, pressure sensing wire lumen 36 may extend to distal portion 32 of elongate shaft 18 to be coupled to a pressure sensor 38, as shown in FIGS. 4-5. In one embodiment, pressure sensor wire lumen 36 may be eliminated wherein a signal from pressure sensor 38 is sent to a computing device 40 other than via a wire 42 in a dedicated pressure sensor wire lumen 36, such as, but not limited to, wireless transmission or integration of wire 42 into the wall of elongate shaft 18. In other embodiments of an elongate shaft or tubular component in accordance herewith, pressure sensor wire lumen 36 may be eliminated wherein the shaft or a portion thereof may be formed by a tubular polymeric inner liner overlaid with a power lead layer and a polymeric outer jacket. In such an embodiment, the power leads for the respective pressure sensor of the inner shaft may be wrapped around the respective shaft for all or at least a portion of the shaft and secured in position by the polymeric outer jacket so as to be embedded within the shaft. In another such embodiment, the power lead for the respective pressure sensor of the inner shaft may be straight for a section or for the entire length of the shaft, and secured in position against the inner liner by the polymeric outer jacket so as to be embedded within the shaft.

Distal portion 32 of elongate shaft 18 is configured to receive a guidewire 44 in a distal portion of guidewire lumen 35 thereof. Further, as shown in FIGS. 1, distal portion 32 is sized to extend from a proximal side 46 of lesion 16, through lesion 16, and to a distal side 48 of lesion 16 such that distal tip 33 is disposed on distal side 48 of lesion 16. Accordingly, in an embodiment, distal portion 32 has a length L_{D} in the range of 25-300 mm. However, length L_{D} may be any length suitable such that distal portion 32 may extend from proximal side 46 to distal side 48. Further, because distal portion 32 is configured to extend through lesion 16, the cross-sectional dimension or profile of distal portion 32 is minimized such as to minimize the disruption of blood flow through lesion 16 in order to obtain an accurate FFR measurement.

Distal tip 33 is disposed on distal portion 32 of elongate shaft 18. In an optional embodiment (not shown), distal tip 33 is disposed on intermediate portion 30 of elongate shaft 18 and is located proximally of distal portion 32. Distal tip 33 includes pressure sensor 38 for measuring a pressure of a fluid within lumen 12 of vessel 14, as shown in FIG. 4. In the embodiment shown in FIG. 4, pressure sensor 38 is disposed in a pocket 50 of a thickened portion 52 of distal tip 33. As shown in FIGS. 4, pocket 50 may be defined by at least one substantially vertical sidewall 54 and substantially horizontal shaft wall 34. In another embodiment, pocket 50 has at least one sidewall with a curvilinear shape. Pressure sensor 38 may be a piezo-resistive pressure sensor, a piezo-electric pressure sensor, a capacitive pressure sensor, an electromagnetic pressure sensor, an optical pressure sensor, and/or combinations thereof. In one non-limiting example, pressure sensor 38 is a micro electromechanical sensor (MEMS) based pressure die measuring about 240 microns by 70 microns by 1100 microns in size. However, other sized pressure sensors may be used. As shown in FIG. 2, thickened portion 52 needs to accommodate pressure sensor 38. Accordingly, thickened portion 52 of elongate shaft 18 causes tip portion 33 to have an outer diameter OD₁ (shown in Fig 2) which is larger than the outer diameter OD₂ of distal portion 32 of elongate shaft 18. However, depending on the size of pressure sensor 38, the outer diameters OD₁ and OD₂ of the elongate shaft 18 could have substantially the same diameter. In one embodiment, outer diameter OD₁ of tip portion 33 is in the range of 0.024 inch - 0.040 inch in order to accommodate pressure sensor 38. However, outer diameter OD₁ may vary depending on the size of pressure sensor 38, thickness of elongate shaft 18, and other factors used to determine the diameter or profile of shafts. In an optional embodiment, a cover (not shown) could extend substantially over pocket 50 to protect pressure sensor 38 from contacting the vessel wall while still allowing blood flow to surround pressure sensor 38.

Pocket 50 is in communication with pressure sensor wire lumen 36 such that any communication wire(s) 42 from pressure sensor 38 may extend from pocket 50 proximally through pressure sensor wire lumen 36, through a corresponding lumen in luer fitting 22 exiting through proximal port 53 to a computing device 40 coupled to proximal end 56 of communication wire 42. Proximal end 56 of communication wire 42 may be coupled to computing device 40 via various communication pathways, including but not limited to one or more physical connections including electrical, optical, and/or fluid connections, a wireless connection, and/or combinations thereof. Accordingly, it is understood that additional components (e.g., cables, connectors, antennas, routers, switches, etc.) not illustrated in FIG. 1 may be included to facilitate communication between the proximal end 56 of communication wire 42 and computing device 40. In an optional embodiment, computing device 40 is incorporated into catheter 10 or for example, in proximal portion 28.

FIG. 4 is a cross-sectional view of distal tip 33. Therein, sensor 38 has a first surface 60, second surface 62, first end 64 and a second end 66. A diaphragm 58 of sensor 38 is disposed on first surface 60 but in other embodiments, diaphragm 58 cold be disposed anywhere on sensor 38. Communication wires 42 (for example, 0.0025 inch coated copper wire in a trifilar configuration) extending from lumen 36 are coupled to an electrical interface, such as an interposer 70 which has first and second surfaces 72, 74. In this embodiment, communication wires form an "S-shape", such that one end of the communication wires 42 is raised up to the elevated level of first surface 72 of interposer 70. Second sensor surface 62 is coupled to first surface 72 of interposer 70 (for example, by an adhesive 76), thereby disposing interposer 70 between shaft wall 34 of elongate shaft 18 and sensor 38.

Sensor wires 80 (for example, 0.001 inch gold wires) have a first end coupled to first surface 72 of interposer 70 and a second end coupled to first surface 60 of sensor 38 adjacent to first end 64. Similarly to the communication wires, sensor wires may also make an S-shape, such that one end of the sensor wires 80 is raised up to the elevated level of first surface 60 of sensor 38. Interposer 70 has second surface 74 coupled to shaft wall 34 of elongate shaft 18. In one embodiment, interposer 70 is coupled to shaft wall 34 by an adhesive 82 having a layer depth of about 25 microns. Sensor 38 may be elevated above shaft wall 34 by the thickness of interposer 70 and to some degree by the thickness of the adhesive layers 76 and 82.

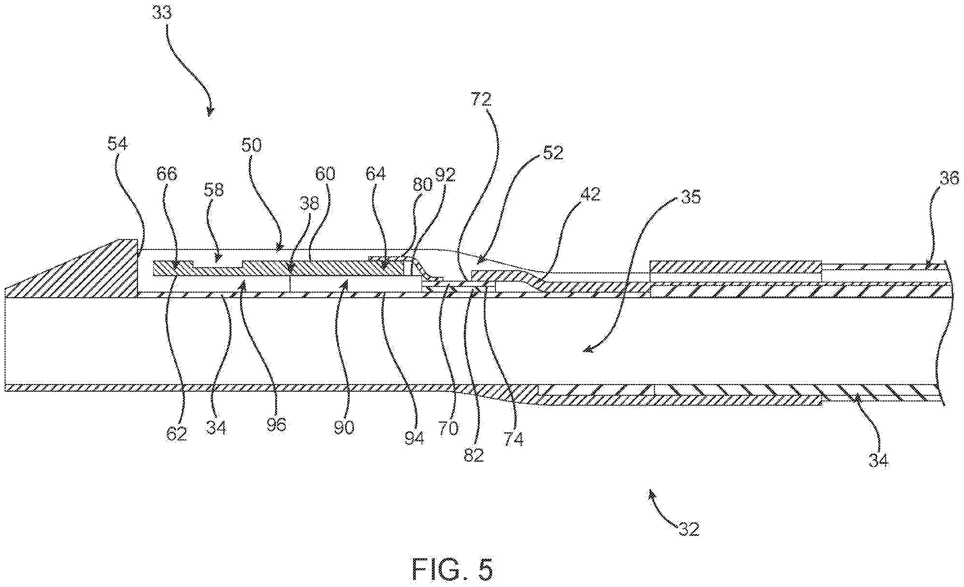

FIG. 5 is a cross-sectional view of another embodiment of distal tip 33. Instead of sensor 38 being mounted on interposer 70 as in FIG. 4, sensor 38 is elevated above shaft wall 34 by a deformable member 90 disposed between sensor 38 and shaft wall 34. Deformable member 90 has a first surface 92 and a second surface 94. First surface 92 of deformable member 90 is coupled to sensor 38 along second surface 62 and second surface 94 is coupled to shaft wall 34. In one embodiment, deformable member 90 is any material having a lower durometer than shaft wall 34 in order to minimize the transfer of stress and strain being experienced by shaft wall. In one embodiment, deformable member 90 is coupled to sensor 38 and shaft wall 34 by, for example, an adhesive. However, in another embodiment, deformable member 90 comprises silicone with adhesive properties.

Deformable member 90 elevates sensor 38 above shaft wall 34 by a distance, for example, of about 40-50 microns. In another example, the amount of distance between the sensor 38 and shaft wall 34 is about 25-60 microns. As shown in FIG. 5, sensor 38 is coupled to deformable member 90 at a location that is adjacent first end 64 of sensor 38. Placement of deformable member 90 at this location with respect to sensor 38 creates a pocket 96 under first end 64 of sensor 38, such that first end 64 of sensor 38 is spaced apart from shaft wall 34. In another embodiment, deformable member 90 can extend along any length of second surface 62 of sensor 38. For example, deformable member 90 can extend along substantially the entire length of second surface 62 of sensor 38 such that second end 62 of sensor is not suspended above shaft wall 34. In yet another embodiment, deformable member 90 has a recess (not shown) formed in first surface 92 which is sized to receive sensor 38 therein.

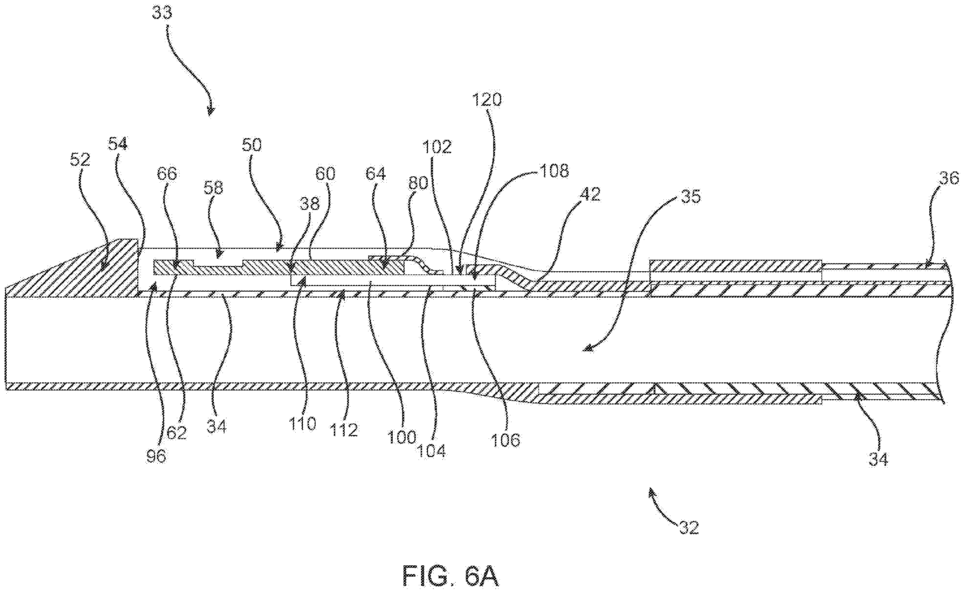

FIG. 6A is a cross-sectional view of another embodiment of distal tip 33. Instead of sensor 38 being mounted on interposer 70 as in FIG. 4, sensor 38 is elevated above shaft wall 34 by an intermediate member 100 disposed between sensor 38 and shaft wall 34. Intermediate member 100 has a first surface 102 and a second surface 104. First surface 102 of intermediate member 100 is coupled to sensor 38 along second surface 62 and second surface 104 is coupled to shaft wall 34 by, for example, an adhesive 106. More specifically, as shown in FIG. 6A, intermediate member 100 has a first portion 108 and a second portion 110. First portion 108 is coupled to shaft wall 34 via adhesive 106, while sensor 38 is coupled to second portion 110 along first surface 102. Although FIG. 6A shows first end 64 of sensor 38 coupled to intermediate member 100 with intermediate member 100 extending approximately halfway across second surface 62, intermediate member 100 could extend along any length of second surface 62 of sensor 38.

With first portion 108 of intermediate member 100 coupled to shaft wall 34 by, for example, adhesive 106, intermediate member 100 is elevated above shaft wall forming a second pocket 112. Thus, sensor 38 would be spaced apart from shaft wall 34 by pocket 96, and intermediate member 100 would be spaced apart from shaft wall by second pocket 112, thereby further isolating sensor 38 from the bending stresses and strains of shaft wall 34. In another embodiment, second pocket 112 is eliminated, and instead, second surface 104 of intermediate member 100 extends along shaft wall 34. In the same embodiment, first surface 102 of intermediate member 100 extends along any length of second surface 62 of sensor 38, such as, for example, intermediate member 100 extending approximately halfway across second surface 62 or intermediate member 100 extending along the entire length of second surface 62.

Intermediate member 100 has a hinge 120 on first portion 108. In one embodiment, hinge 120 is a channel on first or second surfaces 102, 104 (or on both first and second surfaces 102, 104) extending transverse to a longitudinal axis of intermediate member 100. In this embodiment, hinge 120 can have a plurality of cross sectional profile shapes, such as rectangular, arcuate or triangular. In other embodiments, hinge 120 can be a living hinge or any type of hinge that allows two portions of intermediate member 100 to rotate relative to each other about a fixed axis of rotation.

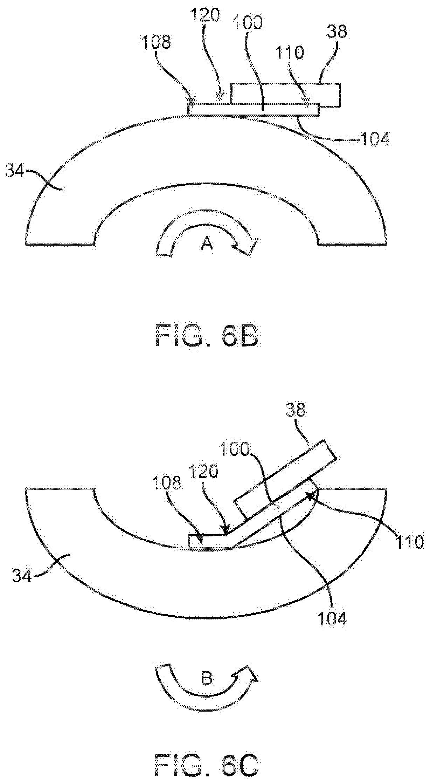

FIGS. 6B-C are exemplary illustrations of shaft wall 34 under bending stress and strain. FIGS. 6B-C are exaggerated to better show the functionality of hinge 120. FIG. 6B shows shaft wall 34 bending or flexing in the direction of arrow A. As can be seen in FIG. 6B, intermediate member 100 remains substantially straight despite shaft wall 34 bending away from intermediate member 100. As a result, sensor 38 also remains substantially straight and is not affected by the bending of shaft wall 34 in the direction of arrow A. In one embodiment, intermediate member 100 is at least as rigid as shaft wall 34. In another embodiment, intermediate member 100 is less rigid that shaft wall 34. However, in either embodiment, intermediate member 100 must be at least rigid enough to maintain sensor in a substantially straight position relative to shaft wall 34.

As shown in FIG. 6C, shaft wall 34 is bending or flexing in the direction of arrow B causing second portion 110 of intermediate member 100 to move in a direction away from shaft wall 34. Specifically, second portion 110 rotates about an axis of rotation provided by hinge 120, wherein the axis of rotation is substantially perpendicular to the longitudinal axis of intermediate member 100. As second portion 110 is rotating about hinge 120, first portion 108 of intermediate member 100 remains coupled to shaft wall 34. Since sensor 38 is coupled to second portion 110 of intermediate member 100, sensor 38 also is rotated about hinge 120. In this way, sensor will maintain being spaced apart from shaft wall 34 thereby preventing stress and strain from being transmitted to sensor 38.

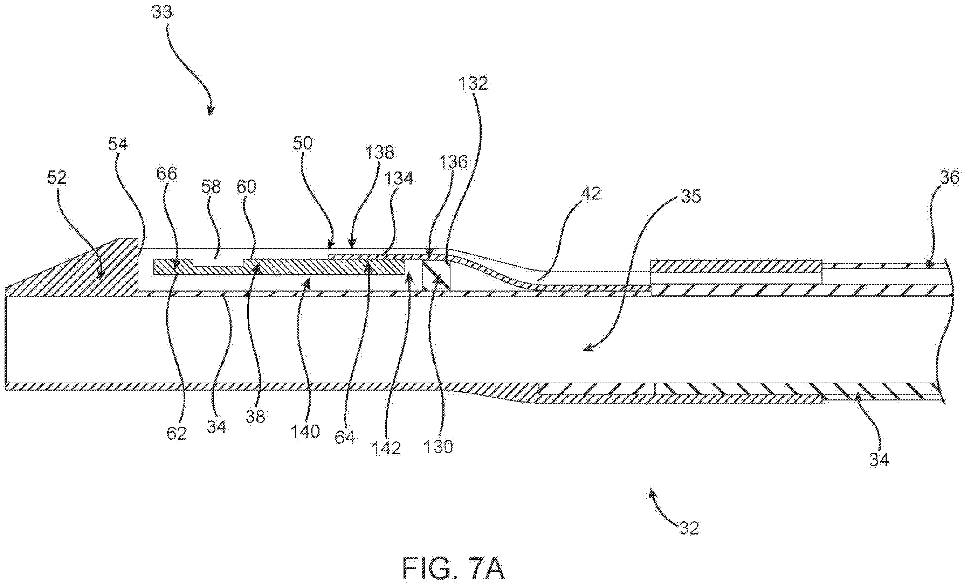

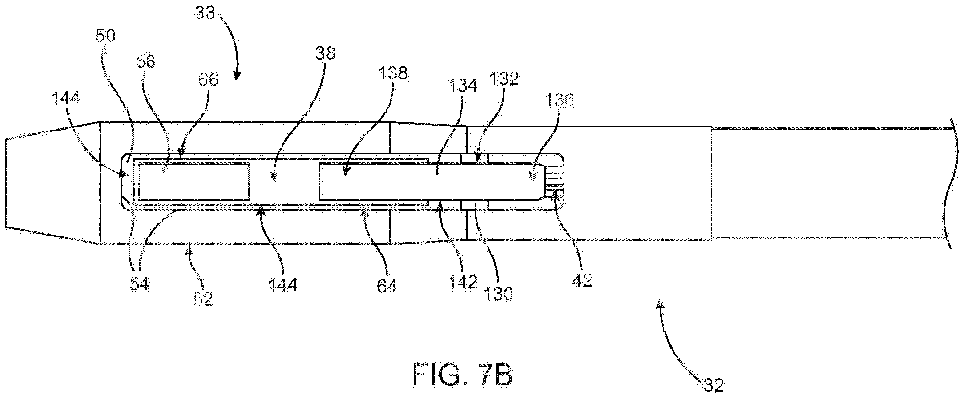

FIG. 7A is a cross-sectional view of another embodiment of distal tip 33. FIG. 7B is a top view of distal tip 33 of FIG. 7A. In the embodiment of FIGS. 7A-B, sensor is not mounted to shaft wall 34 but is instead suspended above shaft wall 34 by a step 130. Step 130 extends from shaft wall 34 and has a top surface 132. Communication wiring 42 (or other electrical wiring) or an electrical interconnect 134 is disposed on top surface 132 of step 130. As shown in FIG. 7A, second surface 60 of sensor 38 is coupled to electrical interconnect 134. Specifically, electrical interconnect 134 has a first portion 136 and a second portion 138. First portion 136 of electrical interconnect 134 is coupled to step 130 while second portion 138 is disposed within pocket 50 without contacting shaft wall 34 or sidewalls 54. As shown in FIG. 7A, sensor 38 is coupled to second portion 138 of electrical interconnect 100. In this configuration, sensor 38 is suspended within pocket 50 and therefore does not contact shaft wall 34 or sidewalls 54. The suspended sensor 38 being spaced apart from shaft wall 34 defines a first pocket 140. As shown in FIG. 7A, first end 64 of sensor 38 is spaced apart from step 130 to form a second pocket 142 between sensor 38 and step 130. As shown in FIG. 7B, sensor 38 is spaced apart from side walls 54 to define a third pocket 144 between sensor 38 and side walls 54. Thus, third pocket extends around the outer periphery of sensor 38 as shown in FIG 7B.

In order to suspend sensor 38 within pocket 50, electrical interconnect 134 needs to be rigid enough to support the weight of sensor 38 and maintain the sensor 38 in a substantially straight configuration relative to shaft wall 34, so as to prevent sensor 38 from contacting shaft wall 34. This is especially the case when shaft wall 34 experiences bending or flexing forces. To better maintain the stability of suspended sensor 38 during bending or flexing, in an optional embodiment, step 130 has grooves or channels (not shown) formed into top surface 132 to receive electrical interconnect 134 (or other electrical or communication wires).

A method of measuring FFR using measurement catheter 10 will now be described with reference to FIG 1. As would be understood by those skilled in the art, when measuring FFR a guide catheter (not shown) may be advanced through the vasculature such that the guide catheter is disposed within the aorta with a distal end thereof disposed within the aorta at an ostium of the aorta adjacent the branch vessel 14 within which lesion 16 is located. As shown in FIG. 1, guidewire 44 can be advanced intraluminally through the guide catheter, into vessel 14 within lumen 12 to the site of lesion 16. In the embodiment shown, guidewire 44 is advanced from proximal side 46 of lesion 16 to distal side 48 of lesion 16, which is also consistent with the direction of the blood flow BF, as indicated by the arrow BF in FIG. 1. In an embodiment, vessel 14 is a coronary artery, but vessel 14 may be other vessels in which it may be desirable to measure pressure, and in particular, to measure FFR.

Thereafter, as shown in FIG. 1, measurement catheter 10 can be tracked or advanced over indwelling guidewire 44 to the target site such that distal end 32 of elongate shaft 18 is positioned distal of lesion 48. As can be seen in FIG. 1, distal tip 33 including pressure sensor 33 can be disposed distally of lesion 16 such that elongate shaft 18 is disposed through lesion 16.

With measurement catheter 10 in place, pressure sensor 33 measures the pressure of blood distal of the lesion within lumen 12. Accordingly, the pressure measured by pressure sensor 33 is the distal pressure measurement, or P_{d}, used in calculating FFR. In one embodiment, adenosine is administered either intracoronary at the site, bolus, or intravenously by continuous infusion for providing an accurate distal pressure measurement (P_{d}) for an FFR value. A proximal pressure measurement Pₐ, which is taken in the aorta by an external AO pressure transducer associated with the guide catheter, and a simultaneous pressure measurement Pₐ taken with pressure sensor 33 of measurement catheter 10 are then obtained to provide the FFR value, *i.e.*, P_{d}/Pₐ, for the lesion. The proximal pressure measurement Pₐ and distal pressure measurement P_{d} can be communicated to computing device 40. Computing device 40, shown schematically in FIGS. 1 and 2, may include such components as a CPU, a display device, an amplification and filtering device, an analog-to-digital converter, and various other components. Computing device 40 may receive the proximal pressure measurement Pₐ and distal pressure measurement P_{d}, and may process them to provide a continuous display of FFR measurement.

When the FFR measurement is completed, measurement catheter 10 may then be completely withdrawn from the patient or repositioned *in vivo* at another lesion and the process repeated. Pressure-sensing catheters in accordance with embodiments hereof may be used for other than providing proximal and distal pressure measurements (Pₐ, P_{d}) for calculating an FFR value. For instance, pressure-sensing catheters in accordance with embodiments hereof may be used to provide an in vivo pressure measurement anywhere along the vasculature, or a particular lesion therein. As well, embodiments hereof may be used to provide in vivo pressure measurements, across a heart valve, venous valve or other valvular location within the body where it may be deemed useful.

The detailed description is merely exemplary in nature and is not intended to limit the invention or the application and uses of the invention. Although the description of the invention is in the context of treatment of blood vessels such as the coronary arteries, the invention may also be used in any other body passageways where it is deemed useful such as but not limited to peripheral arteries, carotid arteries, renal arteries, and/or venous applications. Furthermore, there is no intention to be bound by any expressed or implied theory presented in the preceding technical field, background, brief summary or the detailed description.

While various embodiments according to the present invention have been described above, it should be understood that they have been presented by way of illustration and example only, and not limitation. It will be apparent to persons skilled in the relevant art that various changes in form and detail can be made therein without departing from the scope of the invention. Thus, the breadth and scope of the present invention should not be limited by any of the above-described exemplary embodiments. It will also be understood that each feature of each embodiment discussed herein, and of each reference cited herein, can be used in combination with the features of any other embodiment. The invention is defined by the appended claims.

## Claims

1. A catheter (10) comprising:
an elongate shaft (18) including a proximal portion (28) and a distal portion (32), the elongate shaft (18) having a shaft wall (34), the shaft wall having an outer and inner surface, the shaft wall inner surface defining a guidewire lumen (35);
a deformable member (90) coupled to the shaft wall outer surface at the distal end (33) of the elongate shaft (18), wherein the deformable member (90) is a material having a lower durometer than the shaft wall (34); and
a pressure sensor (38) coupled to the deformable member (90), wherein the sensor has first and second surfaces (60, 62) and the sensor is coupled to the deformable member (90) along the second surface (62).

2. The catheter (10) of claim 1, wherein the deformable member (90) extends substantially along the entire length of second surface of sensor.

3. The catheter (10) of claim 1, wherein the sensor has one portion coupled to the deformable member (90) such that another portion of the sensor defines a pocket (96) between the sensor and the shaft wall (34).

4. The catheter (10) of claim 1, wherein the deformable member (90) has a top surface with a recess formed therein to receive the sensor or wherein the deformable member (90) has adhesive properties.

5. A catheter (10) comprising:
an elongate shaft (18) including a proximal portion and a distal portion, the elongate shaft (18) having a shaft wall (34), the shaft wall (34) having an outer and inner surface, the shaft wall inner surface defining a guidewire lumen (35);
an intermediate member (100) having a first and second portion (108, 110), wherein the first portion (108) is coupled to the shaft wall outer surface at the distal end of the elongate shaft (18) such that the intermediate member (100) is elevated above the shaft wall (34), the intermediate member (100) having a hinge (120) disposed between the first and second portion (108, 110) such that the second portion (110) is rotatable about an axis of rotation defined by the hinge (120), the axis of rotation is traverse to a longitudinal axis of the intermediate member (100); and
a pressure sensor (38) coupled to the second portion (110) of the intermediate member (100).

6. The catheter (10) of claim 5, wherein the second portion (110) of the intermediate member (100) is rotated about the axis of rotation by bending forces from the shaft wall (34) which are a result from when the elongate shaft (18) is tracked to a treatment site within a vasculature.

7. The catheter (10) of claim 5, wherein the intermediate member (100) is sufficiently rigid to maintain the sensor in a substantially straight configuration relative to the shaft when the shaft wall (34) bends away from the intermediate member (100) as a result of bending forces from when the elongate shaft (18) is tracked to a treatment site within a vasculature.

8. The catheter (10) of claim 5, wherein the axis of rotation is substantially perpendicular to the longitudinal axis.

9. The catheter (10) of claim 5, wherein the hinge (120) is disposed on at least one of a first and second surface of the intermediate member (100).

10. The catheter (10) of claim 5, wherein the intermediate member (100) is sufficiently rigid to maintain the sensor in a substantially straight configuration relative to the shaft when the shaft wall (34) bends away from the intermediate member (100) as a result of bending forces from when the elongate shaft (18) is tracked to a treatment site within a vasculature.

11. A catheter comprising:
an elongate shaft (18) including a proximal portion (28) and a distal portion (32), the elongate shaft (18) having a shaft wall (34), the shaft wall having an outer and inner surface, the shaft wall inner surface defining a guidewire lumen (35), further wherein the shaft wall (34) defines a sensor wire lumen (36);
a step (130) extending from the shaft wall outer surface at the distal end of the elongate shaft (18), the step (130) having a top surface;
wherein a sensor wire (80) is disposed on the top surface of the step (130) with a first portion extending distally past the step (130) and a second portion extending proximally through the pressure sensor wire lumen; and
a pressure sensor (38) coupled to the first portion of the sensor wire such that the sensor is spaced apart from the shaft wall (34) to define a first pocket therebetween, and
further comprising a pocket defined by the shaft wall (34) and side walls, the pocket formed on the distal end of the elongate shaft (18), wherein the sensor is suspended within the pocket such that the sensor does not contact the side walls of the pocket.

12. The catheter (10) of claim 11, wherein the sensor wire is an electrical interconnect or wherein the top surface has recesses to receive the sensor wire.

13. The catheter (10) of claim 11, wherein the sensor is spaced apart from the step (130) to define a second pocket and spaced apart from the side walls to define a third pocket, wherein the third pocket extends around the outer periphery of the sensor.

## Patentansprüche

1. Katheter (10), umfassend:
einen länglichen Schaft (18), der einen proximalen Abschnitt (28) und einen distalen Abschnitt (32) einschließt, wobei der längliche Schaft (18) eine Schaftwand (34) aufweist, wobei die Schaftwand eine Außenoberfläche und eine Innenoberfläche aufweist, wobei die Innenoberfläche der Schaftwand ein Führungsdrahtlumen (35) definiert;
ein verformbares Element (90), das an die Außenoberfläche der Schaftwand am distalen Ende (33) des länglichen Schafts (18) gekoppelt ist, wobei das verformbare Element (90) ein Material mit einem niedrigeren Durometerwert als die Schaftwand (34) ist; und
einen Drucksensor (38), der an das verformbare Element (90) gekoppelt ist, wobei der Sensor erste und zweite Oberflächen (60, 62) aufweist und der Sensor entlang der zweiten Oberfläche (62) an das verformbare Element (90) gekoppelt ist.

2. Katheter (10) nach Anspruch 1, wobei das verformbare Element (90) sich im Wesentlichen entlang der gesamten Länge der zweiten Oberfläche des Sensors erstreckt.

3. Katheter (10) nach Anspruch 1, wobei der Sensor einen Abschnitt aufweist, der an das verformbare Element (90) gekoppelt ist, so dass ein anderer Abschnitt des Sensors eine Tasche (96) zwischen dem Sensor und der Schaftwand (34) definiert.

4. Katheter (10) nach Anspruch 1, wobei das verformbare Element (90) eine Deckoberfläche mit einer darin gebildeten Aussparung aufweist, um den Sensor aufzunehmen, oder wobei das verformbare Element (90) Klebeeigenschaften hat.

5. Katheter (10), umfassend:
einen länglichen Schaft (18), der einen proximalen Abschnitt und einen distalen Abschnitt einschließt, wobei der längliche Schaft (18) eine Schaftwand (34) aufweist, wobei die Schaftwand (34) eine Außenoberfläche und eine Innenoberfläche aufweist, wobei die Innenoberfläche der Schaftwand ein Führungsdrahtlumen (35) definiert;
ein Zwischenelement (100) mit einem ersten und einem zweiten Abschnitt (108, 110), wobei der erste Abschnitt (108) an die Außenoberfläche der Schaftwand an dem distalen Ende des länglichen Schafts (18) gekoppelt ist, so dass das Zwischenelement (100) über die Schaftwand (34) erhaben ist, das Zwischenelement (100) ein Scharnier (120) aufweist, das zwischen dem ersten und dem zweiten Abschnitt (108, 110) angeordnet ist, so dass der zweite Abschnitt (110) um eine Rotationsachse rotierbar ist, die durch das Scharnier (120) definiert ist, wobei die Rotationsachse quer zu einer Längsachse des Zwischenelements (100) verläuft; und
einen Drucksensor (38), der an den zweiten Abschnitt (110) des Zwischenelements (100) gekoppelt ist.

6. Katheter (10) nach Anspruch 5, wobei der zweite Abschnitt (110) des Zwischenelements (100) durch Biegekräfte von der Schaftwand (34), die ein Resultat davon sind, dass der längliche Schaft (18) innerhalb eines Gefäßsystems zu einem Behandlungssitus zieht, um die Rotationsachse rotiert.

7. Katheter (10) nach Anspruch 5, wobei das Zwischenelement (100) ausreichend starr ist, um den Sensor relativ zu dem Schaft in einer im Wesentlichen geraden Konfiguration zu halten, wenn die Schaftwand (34) als Resultat von Biegekräften, wenn der längliche Schaft (18) innerhalb eines Gefäßsystems zu einem Behandlungssitus zieht, von dem Zwischenelement (100) weg gebogen wird.

8. Katheter (10) nach Anspruch 5, wobei die Rotationsachse im Wesentlichen rechtwinklig zu der Längsachse ist.

9. Katheter (10) nach Anspruch 5, wobei das Scharnier (120) auf mindestens einer von einer ersten und einer zweiten Oberfläche des Zwischenelements (100) angeordnet ist.

10. Katheter (10) nach Anspruch 5, wobei das Zwischenelement (100) ausreichend starr ist, um den Sensor relativ zu dem Schaft in einer im Wesentlichen geraden Konfiguration zu halten, wenn die Schaftwand (34) als Resultat von Biegekräften, wenn der längliche Schaft (18) innerhalb eines Gefäßsystems zu einem Behandlungssitus zieht, von dem Zwischenelement (100) weg gebogen wird.

11. Katheter, umfassend:
einen länglichen Schaft (18), der einen proximalen Abschnitt (28) und einen distalen Abschnitt (32) einschließt, wobei der längliche Schaft (18) eine Schaftwand (34) aufweist, wobei die Schaftwand eine Außenoberfläche und eine Innenoberfläche aufweist, wobei die Innenoberfläche der Schaftwand ein Führungsdrahtlumen (35) definiert, wobei des Weiteren die Schaftwand (34) ein Sensordrahtlumen (36) definiert;
eine Stufe (130), die sich von der Außenoberfläche der Schaftwand an dem distalen Ende des länglichen Schafts (18) erstreckt, wobei die Stufe (130) eine Deckoberfläche aufweist;
wobei ein Sensordraht (80) auf der Deckoberfläche der Stufe (130) angeordnet ist, wobei sich ein erster Abschnitt distal über die Stufe (130) hinaus erstreckt und ein zweiter Abschnitt proximal durch das Drahtlumen des Drucksensors hindurch erstreckt; und
einen Drucksensor (38), der an den ersten Abschnitt des Sensordrahts gekoppelt ist, so dass der Sensor von der Schaftwand (34) beabstandet ist, um eine erste Tasche dazwischen zu definieren, und
des Weiteren umfassend eine Tasche, die durch die Schaftwand (34) und Seitenwände definiert ist, wobei die Tasche auf dem distalen Ende des länglichen Schafts (18) gebildet ist, wobei der Sensor innerhalb der Tasche aufgehängt ist, so dass der Sensor die Seitenwände der Tasche nicht kontaktiert.

12. Katheter (10) nach Anspruch 11, wobei der Sensordraht eine elektrische Zusammenschaltung ist, oder wobei die Deckoberfläche Aussparungen aufweist, um den Sensordraht aufzunehmen.

13. Katheter (10) nach Anspruch 11, wobei der Sensor von der Stufe (130) beabstandet ist, um eine zweite Tasche zu definieren, und von den Seitenwänden beabstandet ist, um eine dritte Tasche zu definieren, wobei die dritte Tasche sich um die äußere Peripherie des Sensors herum erstreckt.

## Revendications

1. Cathéter (10) comprenant :
un arbre allongé (18) comprenant une partie proximale (28) et une partie distale (32), l'arbre allongé (18) présentant une paroi d'arbre (34), la paroi d'arbre présentant une surface externe et interne, la surface interne de la paroi d'arbre définissant une lumière de fil-guide (35) ;
un élément déformable (90) accouplé à la surface externe de la paroi d'arbre au niveau de l'extrémité distale (33) de l'arbre allongé (18), dans lequel l'élément déformable (90) est un matériau présentant une dureté au duromètre inférieure à celle de la paroi d'arbre (34) ; et
un capteur de pression (38) couplé à l'élément déformable (90), dans lequel le capteur présente des première et seconde surfaces (60, 62) et le capteur est couplé à l'élément déformable (90) le long de la seconde surface (62).

2. Cathéter (10) selon la revendication 1, dans lequel l'élément déformable (90) s'étend sensiblement sur toute la longueur de la seconde surface du capteur.

3. Cathéter (10) selon la revendication 1, dans lequel le capteur présente une partie couplée à l'élément déformable (90) de telle sorte qu'une autre partie du capteur définisse une poche (96) entre le capteur et la paroi d'arbre (34).

4. Cathéter (10) selon la revendication 1, dans lequel l'élément déformable (90) présente une surface supérieure dotée d'un renfoncement formé dans celle-ci pour recevoir le capteur ou dans lequel l'élément déformable (90) a des propriétés adhésives.

5. Cathéter (10) comprenant :
un arbre allongé (18) comprenant une partie proximale et une partie distale, l'arbre allongé (18) présentant une paroi d'arbre (34), la paroi d'arbre (34) présentant une surface externe et interne, la surface interne de la paroi d'arbre définissant une lumière de fil-guide (35) ;
un élément intermédiaire (100) présentant une première et une seconde partie (108, 110), dans lequel la première partie (108) est accouplée à la surface externe de la paroi d'arbre au niveau de l'extrémité distale de l'arbre allongé (18) de telle sorte que l'élément intermédiaire (100) soit élevé au-dessus de la paroi d'arbre (34), l'élément intermédiaire (100) présentant une charnière (120) disposée entre la première et la seconde partie (108, 110) de telle sorte que la seconde partie (110) puisse tourner autour d'un axe de rotation défini par la charnière (120), l'axe de rotation étant transversal à un axe longitudinal de l'élément intermédiaire (100) ; et
un capteur de pression (38) couplé à la seconde partie (110) de l'élément intermédiaire (100).

6. Cathéter (10) selon la revendication 5, dans lequel la seconde partie (110) de l'élément intermédiaire (100) est tournée autour de l'axe de rotation par des forces de flexion provenant de la paroi d'arbre (34) qui sont le résultat d'un moment où l'arbre allongé (18) est suivi vers un site de traitement à l'intérieur d'un système vasculaire.

7. Cathéter (10) selon la revendication 5, dans lequel l'élément intermédiaire (100) est suffisamment rigide pour maintenir le capteur dans une configuration sensiblement droite par rapport à l'arbre lorsque la paroi d'arbre (34) fléchit loin de l'élément intermédiaire (100) en résultat à des forces de flexion provenant d'un moment où l'arbre allongé (18) est suivi vers un site de traitement à l'intérieur d'un système vasculaire.

8. Cathéter (10) selon la revendication 5, dans lequel l'axe de rotation est sensiblement perpendiculaire à l'axe longitudinal.

9. Cathéter (10) selon la revendication 5, dans lequel la charnière (120) est disposée sur au moins une d'une première et d'une seconde surface de l'élément intermédiaire (100).

10. Cathéter (10) selon la revendication 5, dans lequel l'élément intermédiaire (100) est suffisamment rigide pour maintenir le capteur dans une configuration sensiblement droite par rapport à l'arbre lorsque la paroi d'arbre (34) fléchit loin de l'élément intermédiaire (100) en résultat à des forces de flexion provenant d'un moment où l'arbre allongé (18) est suivi vers un site de traitement à l'intérieur d'un système vasculaire.

11. Cathéter comprenant :
un arbre allongé (18) comprenant une partie proximale (28) et une partie distale (32), l'arbre allongé (18) présentant une paroi d'arbre (34), la paroi d'arbre présentant une surface externe et interne, la surface interne de la paroi d'arbre définissant une lumière de fil-guide (35), en outre dans lequel la paroi d'arbre (34) définit une lumière de fil de capteur (36) ;
un gradin (130) s'étendant à partir de la surface externe de la paroi d'arbre au niveau de l'extrémité distale de l'arbre allongé (18), le gradin (130) présentant une surface supérieure ;
dans lequel un fil de capteur (80) est disposé sur la surface supérieure du gradin (130) avec une première partie s'étendant distalement au-delà du gradin (130) et une seconde partie s'étendant proximalement à travers la lumière du fil de capteur de pression ; et
un capteur de pression (38) couplé à la première partie du fil du capteur de telle sorte que le capteur soit espacé de la paroi d'arbre (34) pour définir une première poche entre eux, et
en outre comprenant une poche définie par la paroi d'arbre (34) et les parois latérales, la poche étant formée sur l'extrémité distale de l'arbre allongé (18), dans lequel le capteur est suspendu à l'intérieur de la poche de telle sorte que le capteur n'entre pas en contact avec les parois latérales de la poche.

12. Cathéter (10) selon la revendication 11, dans lequel le fil de capteur est une interconnexion électrique ou dans lequel la surface supérieure présente des évidements pour recevoir le fil de capteur.

13. Cathéter (10) selon la revendication 11, dans lequel le capteur est éloigné du gradin (130) pour définir une deuxième poche et éloigné des parois latérales pour définir une troisième poche, dans lequel la troisième poche s'étend autour de la périphérie externe du capteur.
